(19) Europäisches Patentamt
European Patent Office
Office européen des brevets

(11) Publication number: **0 470 774 B1**

# (12) EUROPEAN PATENT SPECIFICATION

(45) Date of publication of patent specification: **23.11.94**

(51) Int. Cl.[5]: **B01J 2/30**, C05G 3/00, C05C 3/00, C07D 295/13

(21) Application number: **91307089.2**

(22) Date of filing: **01.08.91**

## (54) Method for preventing agglomeration of powder.

(30) Priority: **02.08.90 JP 203947/90**
**20.09.90 JP 248679/90**
**20.09.90 JP 248680/90**

(43) Date of publication of application:
**12.02.92 Bulletin 92/07**

(45) Publication of the grant of the patent:
**23.11.94 Bulletin 94/47**

(84) Designated Contracting States:
**AT BE CH DE DK ES FR GB GR IT LI LU NL SE**

(56) References cited:
**EP-A- 0 149 186**
**US-A- 3 364 218**
**US-A- 4 927 931**

**CHEMICAL ABSTRACTS, vol. 86, no. 15, 11th April 1977, page 519, abstract no.106658m, Columbus, Ohio, US; & SU-A-540 866 (T.A. MIKHAILOVA et al.) 30-12-1976**

**CHEMICAL ABSTRACTS, vol. 109, no. 21, 21st November 1988, page 637, abstractno. 189562t, Columbus, Ohio, US; V.S. BORISENKO et al.: "Kinetics and mechanismof the reaction of 1,2-dichloroethane with piperazine"& ZH. OBSHCH. KHIM. 1988, 58(1), 206-10**

**CHEMICAL ABSTRACTS, vol. 102, no. 8, 25th February 1985, page 630, abstract no.71497c, Columbus, Ohio, US; & JP-A-59 127 805 (KONISHIROKU PHOTO INDUSTRY CO., LTD) 23-07-1984**

(73) Proprietor: **Tosoh Corporation**
**4560, Kaisei-cho**
**Shinnanyo-shi, Yamaguchi-ken (JP)**

(72) Inventor: **Onaka, Tadao**
**17-11 Nagata-cho**
**Shinnanyo-shi, Yamaguchi-ken (JP)**

(74) Representative: **Kearney, Kevin David Nicholas et al**
**KILBURN & STRODE**
**30 John Street**
**London, WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to a method for preventing agglomeration of powder which is likely to agglomerate, said method using poly-1,4-alkylenepiperazine as an anti-agglomeration agent.

A powder of an amine such as piperazine or triethylenediamine (hereinafter referred to simply as TEDA) is a compound which usually has coherence and adherence and thus is likely to agglomerate. Not to mention such a specific powder, a highly hygroscopic powder or a highly sublimable powder in general usually readily undergoes agglomeration due to an inclusion of a small amount of moisture or due to an increase of the temperature. Therefore, such an agglomerative powder is required to be handled with due care, and once such a powder has agglomerated, handling tends to be extremely difficult. As measures to prevent agglomeration of such an agglomerative powder, it is common to employ a method of removing impurities contained in the powder and enlarging the particle size of the powder itself, a method of adding an anti-agglomeration agent to the powder or a method of storing the powder in a closed vessel. However, among agglomerative powders, there are those which undergo agglomeration even when stored in a closed vessel, such as piperazine, or a highly sublimable substance such as TEDA for which the tendency to agglomerate increases as the purity is increased. Therefore, there has been no appropriate method for preventing agglomeration of such a powder. Further, such powders have very strong tendencies to agglomerate and it is usually difficult to prevent agglomeration by enlarging the particle size. As a method for preventing agglomeration of such a highly agglomerative powder, it is usually believed to be necessary to incorporate a suitable anti-agglomeration agent.

For the selection of such an anti-agglomeration agent, it is desired to select an agent which is capable of effectively accomplishing the object in an amount as small as possible and which does not impart an odour or a colour to the powder by the addition. Further it is desired to select an anti-agglomeration agent which presents no adverse effects to the physical properties of the powder in connection with the purpose of the powder and which is inexpensive. As conventional anti-agglomeration agents, silica powder (Japanese Unexamined Patent Publication No. 203039/1982) and polyethylene glycols (Japanese Examined Patent Publication No. 46758/1988) are known. However, the silica powder is effective only to temporarily avoid the contact of crystals to one another and its anti-agglomerating action diminishes with time. On the other hand, liquid anti-agglomeration agents such as polyethylene glycols may simply be mixed with TEDA powder. As a consequence, however, the TEDA powder tends to be wet, and long term storage, the liquid tends to flow to the bottom of the container and tends to be non-uniform in the container, whereby the anti-agglomerating action tends to diminish. Further, in either case, the anti-agglomeration agent is required to be added at a relatively high concentration, whereby the purity of TEDA is lowered.

Whereas, Japanese Examined Patent Publications No. 62241/1988 (which corresponds to EP-A-149186 and No. 3142/1989 disclose that by an addition of a TEDA polymer as an additive during a precipitation step, it is possible to simplify the process of the addition so that the process control can be easy, and the TEDA polymer exhibits a high level of anti-agglomerating action, whereby adequate effects can be obtained by an addition of a very small amount of the polymer. However, this TEDA polymer has a high molecular weight and is insoluble in most organic solvents.

On the other hand, as a kind of poly-1,4-ethylenepiperazines, triethylenediamine polymers are known, which are usually synthesized by ring opening cationic polymerization of triethylenediamine. For example, known are a polymerization method using benzene sulfonic acid as ring opening cationic polymerization (H. K. Hall, Journal of Organic Chemistry 28 (1963), 223), a polymerization method using an organic metal or a Friedel-Crafts catalyst or in the absence of a catalyst at a high temperature (Japanese Examined Patent Publication No. 4792/1969) and a polymerization method using a metal halide, an organic acid (picric acid, toluolsulfonic acid, nitric acid, perchloric acid), a halogenated alkyl, an ammonium salt and a quaternary ammonium salt as catalyst (Rasvodovski et al. Journal of Macromol. Science Chemistry, 8 (1974), 242). All the triethylenediamine polymers which are polymerized by the above methods, are light yellow solids and cristallizable, and have an average molecular weight of from 15,000 to 60,000.

Further, it is disclosed that all the polymerizations are conducted under reduced pressure of at most 3 mmHg after purging with an inert gas.

Furthermore, Japanese Examined Patent Publication No. 7607/1989 discloses a process for producing a triethylenediamine polymer having a relative low molecular weight in the presence of sulfuric acid catalyst.

Powders usually have coherence and adherence in many cases. It is common to employ an operation such as granulation or classification to reduce such nature. However, in a case of a agglomerative powder such as highly sublimable powder of e.g. TEDA, sublimation and condensation are repeated due to a change of e.g. the external temperature, whereby a strong bridge will be formed between powder particles (crystals). Thus, TEDA tends to agglomerate entirely in the container and it tends to be difficult to break the

mass down into particles again.

TEDA is usually synthesised or produced from e.g. N-aminoethylpiperazine or hydroxyethylpiperazine. By such a method, TEDA is obtainable as slightly yellow white crystals. As a by-product, an alkylpiperazine or the like is produced. This by-product has some anti-agglomerating action. However, TEDA crystals of high purity have been desired in recent years, and consequently, TEDA crystals having a purity of at least 99.9% are now produced as a result of an improvement in the purification technique. Accordingly, the agglomerating nature of TEDA has been thereby sharply increased, the problem of agglomeration in the production process or during storage has been made worse.

Further, in accordance with convention methods, triethylenediamine polymers which are soluble in water and organic solvents such as alcohols and glycols can not be prepared whichever polymerization conditions are applied. In these conventional methods, various catalysts are required and the polymerization is conducted at high temperature. Furthermore, alkyl piperazines and amine oxides are produced as by-products, whereby purification is made difficult. In addition, according to the ring opening cationic polymerization of triethylenediamine, other poly-1,4-ethylenepiperazines than polyethylenediamine polymers cannot be obtained.

It is an object of the present invention to provide a method for preventing agglomeration of powder having agglomerating nature by using a poly-1,4-ethylenepiperazine, whereby prevention of agglomeration can effectively be conducted by adding a small amount of an anti-agglomeration agent which has no adverse effect to the physical properties of the powder. It is another object of the present invention to provide a process for preparing a poly-1,4-ethylenepiperazine which is soluble in various organic solvents and useful as anti-agglomeration agent at a low temperature without using any catalyst or forming by-products, in an extremely high yield.

As a result of an extensive study in view of the above-mentioned circumstances, the present inventors have found it possible to effectively control agglomeration of powder by using a poly-1,4-ethylenepiperazine as an anti-agglomeration agent.

Furthermore, the inventors have conducted an extensive study of processes for preparing poly-1,4-alkylenepiperazines which are to be used as anti-agglomeration agents in accordance with the present invention. As a result, the present inventors have found it possible to effectively produce poly-1,4-alkylenepiperazines under extremely feasible conditions in a high yield by using piperazine derivatives and dihalogenated alkanes as the starting materials.

Thus, the present invention provides a method for preventing agglomeration of agglomerative powder, characterised in that a poly-1,4-alkylenepiperazine is incorporated as an anti-agglomeration agent to the powder and in that the poly-1,4-alkylenepiperazine is prepared by reacting a piperazine derivative with a dihalogenated alkane.

Now the present invention will be described in detail with reference to the preferred embodiments.

In the present invention, an agglomerative powder is a highly hygroscopic and/or sublimable powder of e.g. piperazine, TEDA, ammonium sulfate, ammonium chloride or sodium chloride.

Two types of causes are conceivable as the main causes for agglomeration of powder i.e. agglomeration due to absorption of moisture and agglomeration due to bridging of powder particles (crystals) by sublimation and condensation. The former can be avoided by packaging. Otherwise it can be avoided by improving the quality control of the product. With respect to the latter, there has been no effective method discovered which presents no adverse effects to the physical properties of the powder and which fully satisfies other conditions. The present invention presents a very effective agglomeration-inhibiting method by incorporating an anti-agglomeration agent which prevents absorption of moisture and the sublimation and condensation action.

The poly-1,4-alkylenepiperazine of the present invention may, for example, be a compound of the following formula (1):

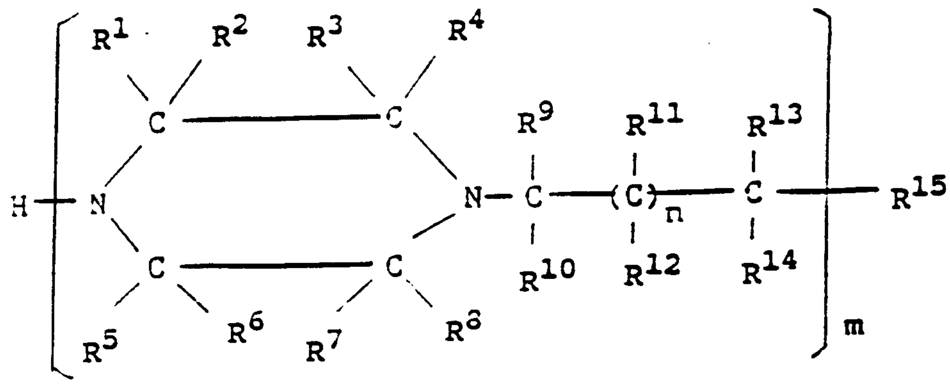

(1)

wherein n is an integer of 0 to 3, m is an integer of at least 1, each of $R^{1-14}$ is H or an alkyl group and $R^{15}$ is a piperazinyl group.

For example, poly-1,4-ethylenepiperazine, poly-1,4-ethylene(2-methyl)piperazine and poly-1,4-(1-methyl)ethylenepiperazine may be mentioned.

According to the present invention, the proportion of the anti-agglomeration agent to the powder having agglomerating nature is not particularly restricted. The amount of addition varies depending on the kind of powder, and it is usually at most 1 part by weight per 100 parts by weight of the powder. It is a feature of the present invention that such a small amount of addition of preferably from 0.001 to 1 part by weight, more preferably from 0.005 to 0.01 part by weight per 100 parts by weight of the powder, attains sufficiently the object of the present invention.

The mechanism for preventing agglomeration in the present invention is considered to be as follows. Poly-1,4-alkylenepiperazines exhibit excellent solubility in various solvents. A solution of the poly-1,4-alkylenepiperazine in water or in an organic solvent is mixed with the powder, followed by drying to form a thin film on the surface of the powder and thereby to microencapsulate the crystals, so that contact of the crystal-forming component with the outer atmosphere in contact of crystals with one another is prevented, whereby the sublimation and condensation are suppressed. Thus, agglomeration of crystals to one another is suppressed, and agglomeration-preventing effects can be obtained.

According to the present invention, there is no particular restriction as to the method for adding the poly-1,4-alkylenepiperazine to the powder. for example, it is common to employ a method wherein after the preparation of powder, the powder and a solution of the poly-1,4-alkylenepiperazine in water or in an organic solvent are thoroughly mixed by means of a mixing apparatus such as a ribbon blender or a V-type mixer. However, to employ such a mixing apparatus, the process tends to be complex, and the cost is expected to be substantial. Whereas, if a solution of the poly-1,4-alkylenepiperazine in water or in an organic solvent is sprayed or otherwise added during the liquid removal step immediately after precipitation i.e. to a TEDA crystal cake in a centrifugal separator, followed by drying, it is possible to effectively and uniformly accomplish the coating on the TEDA crystal surface without requiring any mixing apparatus. Otherwise, it may be added during the precipitation step, as disclosed in Japanese Examined Patent Publication No. 62241/1988.

Anti-agglomeration agents presently used are silica powders and polyethylene glycols. The anti-agglomerating ability of such conventional additives is relatively low, and when the agglomerative powder is TEDA, such conventional additives are required in an amount of about 1 part by weight per 100 parts by weight of TEDA. Whereas, according to the present invention, the poly-1,4-alkylenepiperazine is capable of forming a thin strong film, and a sufficient anti-agglomerating action can be obtained when the poly-1,4-alkylenepiperazine is contained in an amount of at least 0.001 part by weight relative to 100 parts by weight of TEDA. The larger the amount, the higher the anti-agglomerating effects. However, the purity of the powder decreases as the amount of the additive to the powder increases. Therefore, the amount of addition should preferably be as small as possible. According to the present invention, the amount of addition is preferably from 0.001 to 1 part by weight, more preferably from 0.005 to 0.01 part by weight, per 100 parts by weight of the powder. This amount corresponds to from 1/100 to 1/1,000 of the amount of the conventional additives.

Further, the poly-1,4-alkylenepiperazines are chemically very stable. Therefore, they do not adversely affect the physical properties of the powder, and they show excellent solubility in various solvents and thus have excellent properties as additives or coating agents. Now, the process for preparing a poly-1,4-alkylenepiperazine will be described in detail with reference to the preferred embodiments.

In this proess piperazine derivatives according to the following formula (2) are used as starting materials:

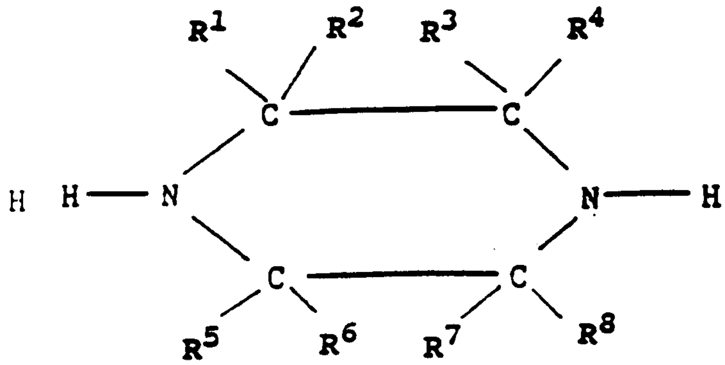

(2)

wherein each of $R^{1-8}$ is H or an alkyl group.

For example, piperazine and 2-methylpiperazine may be mentioned.

Further, the dihalogenated alkane of the present invention is a compound having the following formula (3):

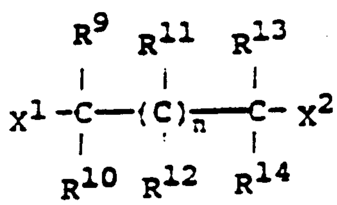

(3)

wherein n is an integer of 0 to 3, each of $R^{9-14}$ is H or an alkyl group and $X^{1-2}$ is a halogen atom.

For example, ethylenedichloride, 1,2-dichloropropane, 1,3-dichloropropane and 1,4-dichlorobutane may be mentioned, which are available as commercial reagents.

Further, the poly-1,4-ethylenepiperazine used in present invention is synthesized from the above two stating materials is for example, a compound of the following formula (1):

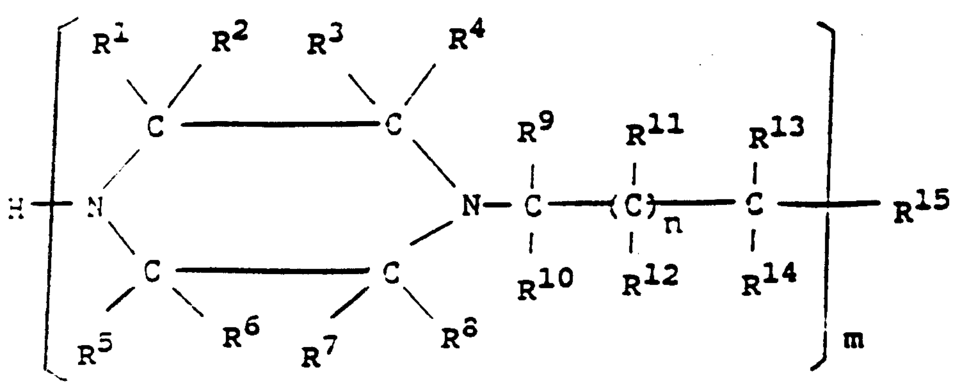

(1)

wherein n is an integer of 0 to 3, m is an integer of at least 1, each of $R^{1-14}$ is H or an alkyl group and $R^{15}$ is a piperazinyl group.

For example, a poly-1,4-ethylenepiperazine, poly-1,4-ethylene(2-methyl)piperazine and poly-1,4-(1-methyl)ethylenepiperazine may be mentioned.

The biggest feature of the present invention resides in that piperazine derivatives and dihalogenated alkanes such as ethylenedichloride are used as the starting materials, whereby poly-1,4-alkylenepiperazines

soluble in various organic solvents can be obtained in an extremely high yield at a low temperature without using any catalyst or forming by-products. Such reaction conditions as used in the present invention are much milder than those of conventional processes. Thus, the process of the present invention is very feasible and poly-1,4-alkylenepiperazines having high purities can be obtained without purification treatment after completion of the reaction.

An excess of a piperazine derivative of the formula (2) may be used with a dihalogenated alkane of the formula (3), and they may be reacted under reflux using water and/or a lower alcohol e.g. methanol or ethanol, as solvent. It is sufficient that the reaction is conducted at a temperature of about from 75 to 85 °C for about 4 to 20 hours. When all the dihalogenated alkane is reacted the liquid temperature begins to rise, which indicates completion of the reaction.

After completion of the reaction, the reaction mixture may be treated with caustic soda. The white substance produced is separated by filtration and washed with a small amount of pure water, followed by drying to obtain a poly-1,4-alkylenepiperazine having a high purity. In this case the yield of the poly-1,4-alkylenepiperazine is substantially in a neutral and acidic solutions, and in organic solvents such as alcohols and glycols.

As described in the foregoing, the present invention employs a poly-1,4-alkylenepiperazine which is soluble in neutral and acidic solutions, and in various solvents such as alcohols and glycols, at a low temperature without using any catalyst in an extremely high yield. The present invention provides an agglomeration-preventing technique in which a very small amount of a poly-1,4-alkylenepiperazine is added to powder to impart excellent agglomeration-preventing effects, it is thought, by suppressing moisture absorption and sublimation of the powder and preventing the contact of the powder particles to one another. The powder particles (crystals) coated can be readily dissolved without the anti-agglomeration agent becoming suspended in the solvent when dissolves.

The invention may be put into practice in various ways and a number of specific embodiments and comparative examples will be described to illustrate the invention with reference to the accompanying drawings.

However, it should be understood that the present invention is by no means restricted by such specific Examples.

## EXAMPLE 1

Into a flask having an internal capacity of 1,000 ml, 86 g of piperazine, 28 g of ethylenedichloride (piperazine/ethylenedichloride > 1 (molar ratio)) and 200 ml of pure water were introduced and refluxed in an oil bath. Here, the liquid temperature was about 83 °C. The reaction was continued until the liquid temperature began to rise. After completion of the reaction, the solution was thoroughly cooled and an excess of caustic soda was added thereto. The formed poly-1,4-ethylenepiperazine precipitated and was collected by filtration after the supernatant liquid was separated. The precipitates were washed with a small amount of pure water an dried under vacuum to obtain 100 g of the poly-1,4-ethylenepiperazine. The yield was 98%. The molecular weight distribution and average molecular weight were from 300 to 1,200 and 600, respectively. The compound exhibited excellent solubility in an aqueous acidic solution and organic solvents such as methanol.

## COMPARATIVE EXAMPLE 1

Into a flask having an internal capacity of 500 ml, 350 g of triethylenediamine, 100 ml of methanol and 0.1 g of a 97% concentrated sulfuric acid (0.0003 mol%) were introduced and heated by a mantle heater. Methanol distilled off was condensed in a cooling tube for recovery. When the liquid temperature had risen to 174 °C, the distillation valve was closed and the solution was reluxed for two hours. Then, the solution was left to cool to 60 °C and methanol was added thereto to dissolve the unreacted monomers. The solution was left to stand to allow the formed triethylenediamine polymers to precipitate. The supernatant liquid was separated and the precipitate was thoroughly washed with methanol, followed by drying to obtain 1.7 g of the triethylenediamine polymer. The yield was 0.5% and the triethylenediamine polymer thus obtained was light yellow and insoluble in water or an organic solvent e.g. methanol, 1,4 butanediol and dipropylene glycol. The molecular weight distribution was from 400 to 7,000 and the average molecular weight was 2,500.

**COMPARATIVE EXAMPLE 2**

The operation was conducted in the same manner as in Comparative Example 1 except that no catalyst was used, whereby 0.1 g of the triethylenediamine polyer was obtained. The yield was 0.03%. The triethylenediamine polymer was light yellow and insoluble in water or an organic solvent e.g. methanol, 1,4 butanediol and dipropylene glycol. The molecular weight distribution was from 400 to 13,000 and the average molecular weight was 3,800.

COMPARATIVE EXAMPLE 3

Into a high pressure vessel having an internal capacity of 100 ml, 10 g of triethylenediamine and 27.8 mg of dihydrobenzene sulfonate were introduced and the vessel was sealed after thoroughly purging with nitrogen. Then, the mixture was heated by a mantle heater and reacted at 200 °C for 10 hours. Then, the reaction product was left to cool to 60 °C and methanol was added thereto to dissolve the unreacted monomers. The solution was left to stand to allow the formed triethylenediamine polymer to precipitate. The supernatant liquid was separated and the precipitate was thoroughly washed with methanol and dried to obtain 9.6 g of the triethylenediamine polymer. The yield was 96%. The molecular weight distribution was from 1,000 to 30,000 and the average molecular weight was 8,800. The polymer was light yellow and adhered on the whole. Further, it was insoluble in an organic solvent e.g. methanol, 1,4 butanediol and dipropylene glycol.

EXAMPLE 2

The operation was conducted in the same manner as in Example 1 except that 100 g of 2-methylpiperazine was used instead of piperazine, whereby 122 g of the poly-1,4-ethylene(2-methyl)-piperazine was obtained. The yield was 98%. The molecular weight distribution was from 400 to 1,200, and the average molecular weight was 600. The polymer exhibited an excellent solubility in an acidic aqueous solution and organic solvents e.g. methanol, 1,4 butanediol and dipropylene glycol.

EXAMPLE 3

The operation was conducted in the same manner as in Example 1 except that 110 g of 1,2-dichloropropane was used instead of ethylenedichloride, whereby 120 g of the poly-1,4-(1-methyl)-ethylenepiperazine was obtained. The yield was 97%. The molecular weight distribution was from 300 to 1,000, and the average molecular weight was 500. The polymer exhibited an excellent solubility in an acidic aqueous solution and organic solvents e.g. methanol, 1,4 butanediol and dipropylene glycol.

EXAMPLE 4

Into a flask having an internal capacity of 1,000 ml, 100 g of piperazine, 100 g of ethylenedichloride and 200 ml of pure water was introduced and reacted in an oil bath under reflux until the liquid temperature began to rise. After completion of the reaction, the solution was thoroughly cooled and an excess of caustic soda was added thereto. The formed poly-1,4-ethylenepiperazine precipitated and was collected by filtration after the supernatant liquid was separated. The precipitate was thoroughly washed with pure water and dried under vacuum to obtain 115 g of the poly-1,4-ethylenepiperazine. The molecular weight distribution was from 400 to 1,000 and the average molecular weight was 500. The polymer exhibited an excellent solubility in an acidic aqueous solution and organic solvents such as methanol, 1,4-butanediol and propylene glycol.

For the test to study the agglomerating nature, TEDA powder having a purity of at least 99.95% was used, and as an anti-agglomeration agent, the soluble poly-1,4-ethylenepiperazine which was prepared above was used. The addition was conducted in the following manner to form a coating of the poly-1,4-ethylenepiperazine on the surface of TEDA.

500 g of a TEDA cake was prepared on a Buechner funnel, and 200 ml of a 0.5% alcohol solution of the above anti-agglomeration agent was added thereto. The mixture was thoroughly mixed to contact the solution with TEDA crystals, followed by filtration to collect the TEDA crystals. The crystals were thoroughly dried under vacuum to obtain 430 g of a sample. The coated amount deposited was 0.03 g (the concentration of the additive in the crystals (same applies hereinafter): 70 ppm).

The measurement of the agglomeration degree and the evaluation standards were as follows. Namely, the obtained sample was packed in a container having a size of 5 cm x 5 cm and a height of 2 cm, and a plastic plate of 5 cm x 5 cm was placed thereon. A weight of 300 g was placed thereon, and the container was stored in a desiccater having a humidity of not higher than 1%. During the storage, the pressure exerted on the crystals was 12 g/$cm^2$.

After storage in the desiccater for one month, the weight and the container were removed, and pressure was exerted to the center portion of the crystal block having the plastic plate located beneath, by a Kiya-type hardness meter, whereby the pressure at breakage was read. The values thus obtained were classified into the following three rankings, which were used as indices for evaluation of the agglomeration degree. In the following Examples and Comparative Examples, the agglomeration degree was evaluated in the same manner. The results are shown in Table 1.

As is evident from Table 1, TEDA treated in this Example belongs to A rank, thus indicating excellent anti-agglomerating effects.

A rank: Crystal block which can readily be broken with a slight impact with a breaking pressure of not higher that 1.0 kg/$cm^2$ and in which no substantial progress of agglomeration was observed.

B rank: Crystal block with a breaking pressure of not higher than 10.0 kg/$cm^2$ which can not be broken by a low level of impact and in which agglomeration was found progressed entirely.

C rank: Crystal block which requires a considerably strong impact for breakage with a breaking pressure of at least 10.0 kg/$cm^2$ and in which agglomeration was found completely progressed.

COMPARATIVE EXAMPLE 4

The operation was conducted in the same manner as in Example 4 except that no anti-agglomeration agent was used.

The results are shown in Table 1.

COMPARATIVE EXAMPLE 5

3,000 ml of a TEDA methanol solution having a composition comprising 50 parts by weight of TEDA and 50 parts by weight of methanol, was introduced into a flask having an internal capacity of 5,000 ml, and 0.15 g of TEDA polymer (prepared by the synthesis disclosed in Japanese Unexamined Patent Publication No. 62241/1988 i.e. cationic polymerization), was added thereto. Here, the TEDA polymer had an average molecular weight of 2,800 and a molecular weight distribution of from 400 to 7,000. The mixture was subjected to methanol removal by an evaporator, whereby 1,100 ml of methanol was distilled. The residual liquid was left to stand still at a room temperature and then cooled to a liquid temperature of 20°C. Precipitated TEDA crystals were collected by filtration under suction with a filter paper of No. 5C and then dried under vacuum to obtain 450 g of TEDA crystals. The TEDA polymer contained in the TEDA crystals was 0.04 g. With respect to this sample, the agglomeration degree was evaluated in the same manner as in Example 4.

The results are shown in Table 1. As is evident from Table 1, excellent agglomeration-preventing effects were exhibited, but when made into a 33 wt% dipropylene glycol solution, a certain level of turbidity was observed.

COMPARATIVE EXAMPLE 6

500 g of TEDA and 2 g of silica gel (manufactured by Nippon Silica Gel Kogyo K.K., bulk density: 40g/ℓ, average particle size: 2 μm) were thoroughly mixed by a V-mixer, and the obtained mixture was used as a sample. Otherwise, the operation was conducted in the same manner as in Example 4. The results are as shown in Table 1, and a certain degree of agglomeration was observed.

COMPARATIVE EXAMPLE 7

500 g of TEDA and 5 g (1%) of polyethylene glycol #200 (manufactured by Kanto Kagaku) were thoroughly mixed by a V-type mixer, and the mixture was used as a sample. Otherwise, the operation was conducted in the same manner as in Example 4. The results are shown in Table 1.

EXAMPLE 5

Using the same poly-1,4-ethylenepiperazine as used in Example 4, the coated amount and the agglomeration-preventing effects were compared. The operation was conducted in the same manner as in Example 4 except that (1) 1%, (2) 0.5%, (3) 0.1%, (4) 0.05% or (5) 0.01% alcohol solution of the soluble poly-1,4-ethylenepiperazine, or (6) pure alcohol, was used.

The amount of TEDA, the coated amount of the poly-1,4-ethylenepiperazine and the agglomeration-preventing action are shown in Table 2. As is evident from Table 2, a certain degree of agglomeration-preventing action was observed even at a coated amount of 10 ppm, and excellent agglomeration-preventing action was observed at a concentration of 70 ppm or higher. Further, no formation of turbidity or floating substance was observed in solutions of various solvents, such as alcohols, water and glycols.

EXAMPLE 6

Into a flask having an internal capacity of 2,000 ml, 1,000 g of an aqueous ammonium chloride solution comprising 35 parts by weight of ammonium chloride and 65 parts by weight of water, was introduced, and 0.5 g of the same soluble poly-1,4-ethylenepiperazine as used in Example 4, was added thereto. The mixture was heated by a mantle heater, and heating was stopped when 300 ml of water was distilled off. The flask was immersed in a water bath and cooled until the liquid temperature became 20 °C. Precipitated ammonium chloride was collected by filtration under suction with a filter paper of No. 5C and washed with a small amount of pure water. The product was dried under vacuum to obtain 92 g of ammonium chloride. Here, the poly-1,4-ethylenepiperazine contained in the crystals was 0.023 g (250 ppm). The agglomeration degree of this sample was evaluated in the same manner as in Example 4. As a result, the agglomeration degree was found to be A rank.

COMPARATIVE EXAMPLE 8

The operation was conducted in the same manner as in Example 6 except that no soluble poly-1,4-ethylenepiperazine was used. In the ammonium chloride thereby obtained, the agglomeration was found progressed to the interior, and the agglomeration degree was found to be C rank.

EXAMPLE 7

Into a flask having an internal capacity of 2,000 ml, 1,000 g of an aqueous ammonium sulfate solution comprising 40 parts by weight of ammonium sulfate and 60 parts by weight of water, was introduced, and 0.5 g of the same poly-1,4-ethylenepiperazine as used in Example 4, was added thereto. The mixture was heated by a mantle heater, and heating was stopped when 400 ml of water was distilled off. The flask was immersed in a water bath and cooled until the liquid temperature became 20 °C. Precipitated ammonium sulfate was collected by filtration under suction with a filter paper of No. 5C and washed with a small amount of pure water. The product was dried under vacuum to obtain 230 g of ammonium sulfate. Here, the soluble poly-1,4-ethylenepiperazine contained in the ammonium sulfate was 0.05 g (217 ppm). The agglomeration degree of this sample was evaluated in the same manner as in Example 4. As a result, the agglomeration degree was found to be A rank.

COMPARATIVE EXAMPLE 9

The operation was conducted in the same manner as in Example 7 except that no soluble poly-1,4-ethylenepiperazine was used. In the ammonium sulfate thereby obtained, agglomeration was found progressed to the interior, and the agglomeration degree was found to be C rank.

EXAMPLE 8

Into a flask having an internal capacity of 1,000 ml, 100 g of 2-methylpiperazine, 100 g of ethylenedichloride and 200 ml of pure water were introduced and reacted in an oil bath under reflux until the liquid temperature began to rise. After completion of the reaction, the solution was thoroughly cooled and an excess of caustic soda was added thereto. The formed poly-1,4-ethylene(2-methyl) piperazine precipitated and was collected by filtration after the supernatant liquid was separated. The precipitate was thoroughly washed with pure water and dried under vacuum to obtain 115 g of the poly-1,4-ethylene(2-methyl)-

piperazine. The molecular weight distribution was from 300 to 1,200 and the average molecular weight was 600. The polymer exhibited an excellent solubility in an acidic aqueous solution and organic solvents such as methanol, 1,4-butanediol and propylene glycol.

By using the poly-1,4-ethylene(2-methyl)piperazine thus obtained, coating was conducted in the same manner as in Example 4. The coated TEDA crystals were thoroughly dried under vacuum to obtain 350 g of a sample. The coated amount was 0.07 g (200 ppm).

With respect to this sample, the agglomeration degree was evaluated in the same manner as in Example 4, and TEDA treated in this Example belongs to A rank, thus indicating excellent anti-agglomerating effects.

EXAMPLE 9

By using the poly-1,4-ethylene(2-methyl)piperazine prepared in Example 8, the coated amount and the agglomeration-preventing effects were compared. The operation was conducted in the same manner as in Example 4 except that (1) 1%, (2) 0.5%, (3) 0.2%, (4) 0.05% alcohol solution of the poly-1,4-ethylene(2-methyl)piperazine, or (5) pure alcohol, was used.

The amount of TEDA, the coated amount of poly-1,4-ethylene(2-methyl)piperazine and the agglomeration-preventing action are shown in Table 3. As is evident from Table 3, a certain degree of agglomeration-preventing action was observed even at a coated amount of 15 ppm, and excellent agglomeration-preventing action was observed at a concentration of 100 ppm or higher.

EXAMPLE 10

The operation was conducted in the same manner as in Example 6 except that the poly-1,4-ethylene(2-methyl)piperazine prepared in Example 8 was used, whereby 88 g of ammonium chloride was obtained. Here, the poly-1,4-ethylenepiperazine contained in the crystals was 0.021 g (239 ppm). The agglomeration degree of this sample was evaluated in the same manner as in Example 4. As a result, the agglomeration degree was found to be A rank.

EXAMPLE 11

The operation was conducted in the same manner as in Example 7 except that the poly-1,4-ethylene(2-methyl)piperazine prepared in Example 8 was used, whereby 210 g of ammonium sulfate was obtained. Here, the poly-1,4-ethylene(2-methyl)piperazine contained in the ammonium sulfate was 0.045 g (214 ppm). The agglomeration degree of this sample was evaluated in the same manner as in Example 4. As a result, the agglomeration degree was found to be A rank.

EXAMPLE 12

The operation was conducted in the same manner as in Example 8 except that piperazine and propylenedichloride were used instead of 2-methylpiperazine and ethylenedichloride, whereby 120 g of the poly-1,4-(1-methyl)ethylenepiperazine was obtained. The molecular weight distribution was from 300 to 1,000, and the average molecular weight was 500.

As a result of coating treatment, 350 g of TEDA was obtained, and the coated amount was 0.07 g (200 ppm). The agglomeration degree was found to be A rank and thus excellent anti-agglomerating effects were recognized.

Table 1

| | Additive | Amount of TEDA (g) | Treating method | Amount added (g) | Concentration of the addit-ive (ppm) | Agglomeration-preventing action | Others |
|---|---|---|---|---|---|---|---|
| Example 1 | Soluble poly-1,4-ethylene-piperazine | 430 | Coating | 0.03 | 70 | A | Transparent when dissolved |
| Comparative Example 1 | Nil | 460 | - | 0 | 0 | C | Coagulation advanced very much |
| Comparative Example 2 | TEDA polymer | 450 | Precipitation | 0.04 | 90 | A | Slight turbidity observed when dissolved |
| Comparative Example 3 | Silica powder | 500 | Mixing | 2 | 4,000 | B | |
| Comparative Example 4 | Polyethylene glycol | 500 | Mixing | 5 | 10,000 | B | Crystals being wet |

Table 2

| No. | Concentration of the additive in the solution (%) | Amount of TEDA (g) | Coated amount (g) | Concentration of the addtive in the powder (ppm) | Agglomeration-preventing action |
|---|---|---|---|---|---|
| S.(1) | 1 | 400 | 100 | 250 | A |
| S.(2) | 0.5 | 430 | 30 | 70 | A |
| S.(3) | 0.1 | 430 | 9 | 21 | B |
| S.(4) | 0.05 | 450 | 4 | 9 | B |
| S.(5) | 0.01 | 430 | 1 | 2 | B |
| S.(6) | 0 | 460 | 0 | 0 | C |

Table 3

| No. | Concentration of the additive in the solution (%) | Amount of TEDA (g) | Coated amount (g) | Concentration of the addtive in the powder (ppm) | Agglomeration-preventing action |
|---|---|---|---|---|---|
| 9.(1) | 1 | 400 | 120 | 300 | A |
| 9.(2) | 0.5 | 380 | 65 | 171 | A |
| 9.(3) | 0.2 | 350 | 22 | 63 | B |
| 9.(4) | 0.05 | 400 | 6 | 15 | B |
| 9.(5) | 0 | 320 | 0 | 0 | C |

## Claims

**1.** A method for preventing agglomeration of powder, wherein a poly-1,4-alkylenepiperazine is incorporated as an anti-agglomeration agent in the powder, characterized in that the poly-1,4-alkylenepiperazine has been prepared by reacting a piperazine derivative with a dihalogenated alkane.

2. A method as claimed in claim 1, characterised in that the said powder is a highly hygroscopic and/or sublimated powder.

3. A method as claimed in claim 1 or claim 2 characterised in that the powder is piperazine, triethylenediamine, ammonium sulfate, ammonium chloride or sodium chloride.

4. A method as claimed in claim 1, 2 or 3 characterised in that the poly-1,4-alkylenepiperazine is a compound of the formula:

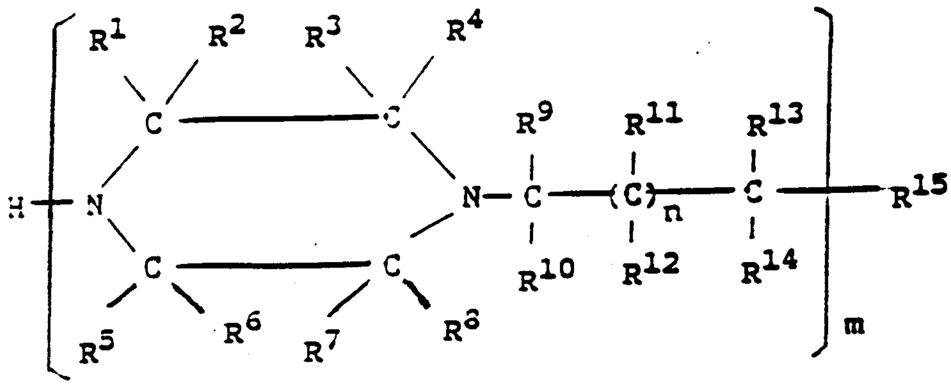

wherein n is an integer of 0 to 3, m is an integer of at least 1, each of $R^{1-14}$ is H or an alkyl group and $R^{15}$ is a piperazyl group.

5. A method as claimed in claim 1, 2, 3 or 4 characterised in that the poly-1,4-alkylenepiperazine is incorporated in an amount of from 0.001 to 1 part by weight per 100 parts by weight of the powder.

6. A method as claimed in any one of claims 1 to 5 characterised in that the piperazine derivative is a compound of the formula:

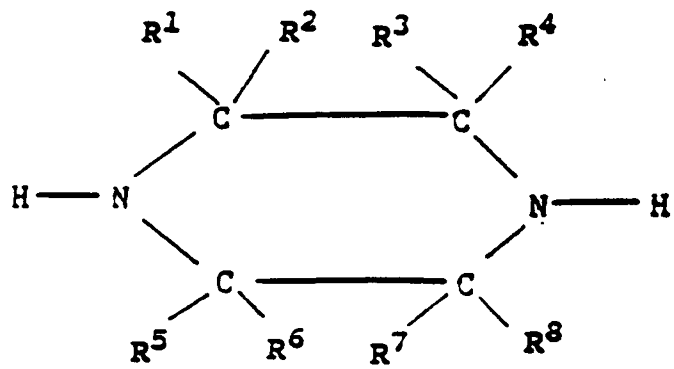

wherein each of $R^{1-8}$ is H or an alkyl group.

7. A method as claimed in any one of claims 1 to 6 characterised in that the dihalogenated alkane is a compound of the formula:

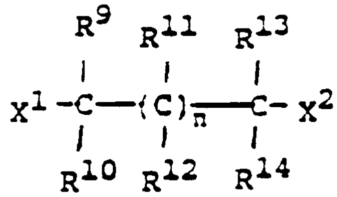

wherein n is an integer of 0 to 3, each of $R^{9-14}$ is H or an alkyl group and each of $X^{1-2}$ is a halogen atom.

**Patentansprüche**

1. Verfahren zur Verhinderung des Zusammenbackens von Pulvern, wobei ein Poly-1,4-alkylenpiperazin als Anti-Agglomeriermittel dem Pulver einverleibt wird, dadurch gekennzeichnet, daß das Poly-1,4-alkylenpiperazin hergestellt wurde durch Umsetzung eines Piperazinderivats mit einem dihalogenierten Alkan.

2. Verfahren gemäß Anspruch 1, dadurch gekennzeichnet, daß das Pulver ein sehr hygroskopisches und/oder sublimierbares Pulver ist.

3. Verfahren gemäß Anspruch 1 oder 2, dadurch gekennzeichnet, daß das Pulver Piperazin, Triethylendiamin, Ammoniumsulfat, Ammoniumchlorid oder Natriumchlorid ist.

4. Verfahren gemäß Anspruch 1, 2 oder 3, dadurch gekennzeichnet, daß das Poly-1,4-alkylenpiperazin eine Verbindung der folgenden Formel ist:

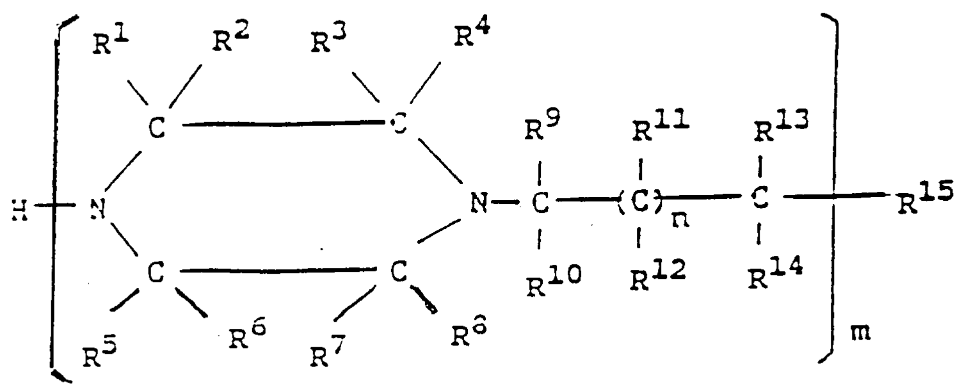

wobei n eine ganze Zahl von 0 bis 3 ist, m eine ganze Zahl von mindestens 1 ist, jedes von $R^{1-14}$ für H oder eine Alkylgruppe steht und $R^{15}$ für eine Piperazylgruppe steht.

5. Verfahren gemäß Anspruch 1, 2, 3 oder 4, dadurch gekennzeichnet, daß das Poly-1,4-alkylenpiperazin in einer Menge von 0,001 bis 1 Gew.-Teil pro 100 Gew.-Teile des Pulvers einverleibt wird.

6. Verfahren gemäß einem der Ansprüche 1 bis 5, dadurch gekennzeichnet, daß das Piperazinderivat eine Verbindung der folgenden Formel ist:

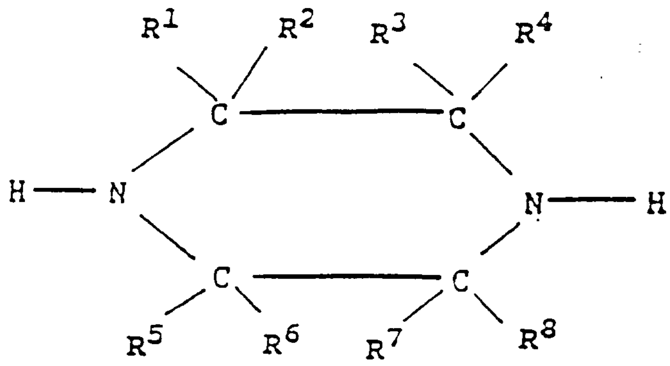

wobei jedes von $R^{1-8}$ für H oder eine Alkylgruppe steht.

7. Verfahren gemäß einem der Ansprüche 1 bis 6, dadurch gekennzeichnet, daß das dihalogenierte Alkan eine Verbindung der folgenden Formel ist:

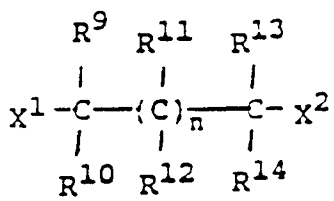

wobei n ein ganze Zahl von 0 bis 3 ist, jedes von $R^{9-14}$ für H oder eine Alkylgruppe steht und jedes von $X^{1-2}$ für ein Halogenatom steht.

## Revendications

1. Procédé pour empêcher l'agglomération d'une poudre, suivant lequel une poly-1,4-alkylènepipérazine est incorporée dans la poudre en tant qu'agent anti-agglomération, caractérisé par le fait que la poly-1,4-alkylènepipérazine a été préparée par réaction d'un dérivé de pipérazine avec un alcane dihalogéné.

2. Procédé selon la revendication 1, caractérisé par le fait que ladite poudre est une poudre hautement hygroscopique et/ou sublimée.

3. Procédé selon la revendication 1 ou la revendication 2, caractérisé par le fait que la poudre est une poudre de pipérazine, de triéthylènediamine, de sulfate d'ammonium, de chlorure d'ammonium ou de chlorure de sodium.

4. Procédé selon la revendication 1, 2 ou 3, caractérisé par le fait que la poly-1,4-alkylènepipérazine est un composé représenté par la formule :

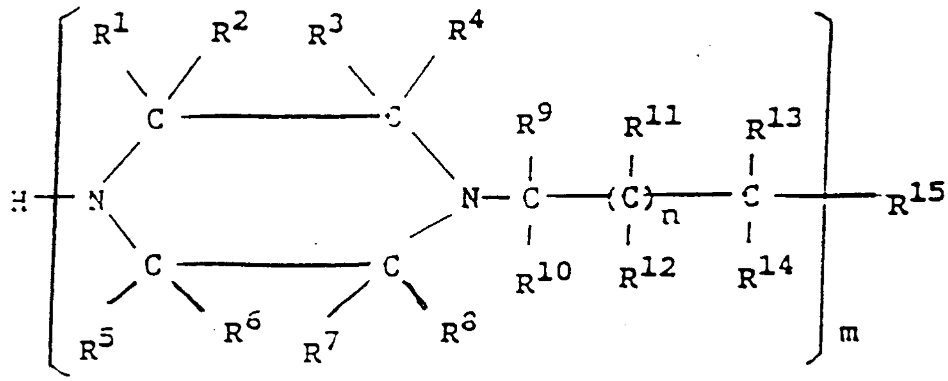

dans laquelle :

- n est un entier de 0 à 3 ;
- m est un entier d'au moins 1 ;
- $R^{1-14}$ représentent chacun H ou un groupe alkyle ; et
- $R^{15}$ représente un groupe pipérazyle.

**5.** Procédé selon la revendication 1, 2, 3 ou 4, caractérisé par le fait que la poly-1,4-alkylènepipérazine est incorporée dans une quantité allant de 0,001 à 1 partie en poids pour 100 parties en poids de la poudre.

**6.** Procédé selon l'une des revendications 1 à 5, caractérisé par le fait que le dérivé de pipérazine est un composé représenté par la formule :

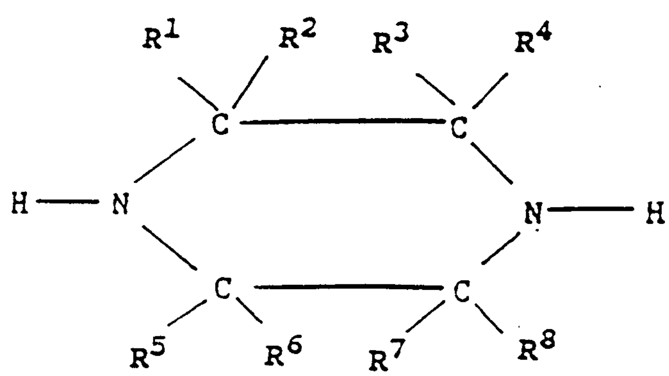

dans laquelle $R^{1-8}$ représentent chacun H ou un groupe alkyle.

**7.** Procédé selon l'une des revendications 1 à 6, caractérisé par le fait que l'alcane dihalogéné est un composé représenté par la formule :

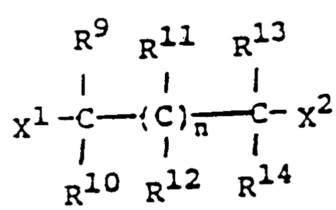

dans laquelle :

- n est un entier de 0 à 3 ;
- $R^{9-14}$ représentent chacun H ou un groupe alkyle ; et

- $X^{1-2}$ représentent chacun un atome d'halogène.